# EUROPÄISCHE PATENTSCHRIFT

(11) **EP 0 460 343 B1**
(45) Veröffentlichungstag und Bekanntmachung des Hinweises auf die Patenterteilung: **13.09.1995**
(21) Anmeldenummer: 90890333.9
(22) Anmeldetag: 17.12.1990
(51) Int. Cl.: G01N 21/11, G01N 1/00

(54) **Einweg-Messelement zur Analyse von gasförmigen oder flüssigen Proben**
Disposable measuring element for analysing liquid or gaseous samples
Elément de mesure à usage unique pour l'analyse d'un échantillon liquide ou gazeux

(30) Priorität: 08.06.1990 AT 1258/90
(43) Veröffentlichungstag der Anmeldung: 11.12.1991
(73) Patentinhaber: AVL Medical Instruments AG, 8207 Schaffhausen (CH)
(72) Erfinder: Karpf, Hellfried, Dr., A-8010 Graz (AT); Leiner, Marco Jean-Pierre, Dr., A-8010 Graz (AT); Möstl, Anton, Dipl.-Ing., A-8055 Graz (AT); Reichenberger, Klaus, Ing., A-8720 Knittelfeld (AT); Schaffar, Bernhard, Mag. Dr., A-8045 Graz (AT); Ziegler, Werner E., Dipl.-Ing., A-8043 Graz (AT)
(74) Vertreter: Krause, Walter, Dr. Dipl.-Ing.

(56) Entgegenhaltungen:
- EP-A- 0 354 895
- US-A- 4 352 780
- US-A- 4 871 439

## Beschreibung

Die Erfindung betrifft ein Einweg-Meßelement, mit einem Meßkanal mit mindestens einem darin angeordneten, optischen oder elektrochemischen Sensor, einer ersten Öffnung an einem Ende des Meßkanals zum Anschluß eines Analysegerätes, einer zweiten Öffnung am anderen Ende des Meßkanals zum Anschluß eines Probennahmeteiles, einem ersten Verschlußorgan für die erste Öffnung und einem zweiten Verschlußorgan für die zweite Öffnung.

Aus der EP-A 354 895 ist ein Einweg-Meßelement zur Analyse von gasförmigen oder flüssigen Proben bekannt. In dem Meßelement ist ein als Kapillare ausgebildeter Meßkanal vorgesehen, der optische Sensoren zur Analyse der Probe enthält. Bis zur Verwendung des Meßelementes ist der Meßkanal an beiden Enden durch eine Membran verschlossen und im Inneren befindet sich ein Lagermedium. Unmittelbar vor der Messung wird die Kalibrierung durchgeführt, wobei das Lagermedium auch als Kalibriermedium dient. Im Anschluß daran wird eine Membran im Bereich eines Probennahmeteiles durchstoßen und die zu analysierende Probe verdrängt das Lagermedium.

Es hat sich nun in der Praxis herausgestellt, daß es äußerst schwierig ist, geeignete Medien zu finden, die sowohl als Lagermedium als auch als Kalibriermedium günstige Eigenschaften aufweisen. Es müssen dabei Kompromisse eingegangen werden, die bei der Verwendung von getrennten Lager- bzw. Kalibriermedien vermieden werden könnten.

Als nachteilig hat sich auch die begrenzte Lagerfähigkeit solcher Meßelemente herausgestellt, da schon durch geringfügige Diffusion die Zusammensetzung des Kalibriermediums verändet wird. Auf diese Weise wird nach längerer Lagerung des Meßelementes die Messung verfälscht.

Aus der US-A 4 871 439 ist in diesem Zusammenhang ein Einweg-Meßelement bekannt geworden, welches zur Analyse von gasförmigen oder flüssigen Proben dient und einen Meßkanal mit mindestens einem optischen oder elektrochemischen Sensor enthält. Der Meßkanal wird an einem Ende durch einen Behälter verschlossen, welcher nach der Messung die Probe sowie die Referenz- und Kalibriermedien aufnehmen kann. Bei der Messung gelangt zuerst eine Kalibrierlösung in den Meßkanal, welche dann durch die Probe - z. B. Blut - verdrängt wird.

Aus der EP-A 122 420 ist eine Elektrodenanordnung zur elektrochemischen Analyse elektrolytischer Bestandteile einer Flüssigkeit bekannt geworden. Gemäß einer Ausführungsvariante weist diese Elektrodenanordnung einen beiderseits mit einer abnehmbaren Kappe verschlossenen Meßkanal zur Aufnahme einer Referenzelektrolytlösung auf. Nach der Kalibriermessung kann eine der Kappen abgenommen und die zu vermessende Probe eingesaugt werden. Der Referenzelektrolyt kann durch Einsaugen einer Luftblase von der Probe getrennt werden.

Schließlich ist aus der US-A 4 654 127 ein Meßelement bekannt geworden, das einen Meßkanal mit mehreren Sensoren aufweist. Dem Eingang des Meßkanals ist ein in zwei Kammern unterteilter Behälter zugeordnet, wobei sich in einer Kammer eine Kalibrierflüssigkeit befindet und in die andere Kammer die zu messende Probe eingefüllt werden kann. Beide Kammern weisen mit durchstechbaren Membranen verschlossene Öffnungen auf, welche mit dem Eingang des Meßkanals in Kontakt gebracht werden können.

Nach dem Eindringen der Probe in die Probenkammer wird - ausgehend von einer Startposition, bei welcher keine der Kammern mit dem Meßkanal in Verbindung steht - der Behälter durch eine Drehbewegung in eine Kalibrierposition gebracht, die den Kalibrierbehälter verschließende Membran durchstoßen und die Kalibrierflüssigkeit durch die im Meßkanal wirkenden Kapillarkräfte in den Meßbereich gesaugt und analysiert. Danach wird durch eine weitere Drehung des Behälters die Probenkammer an den Eingang des Meßkanals herangeführt und nach dem Durchstoßen der Membran die Probe durch dieselbe Öffnung in den Meßkanal eingebracht und ebenfalls analysiert. Aus den erhaltenen Werten der Proben- und Kalibrierflüssigkeit wird auf die zu messenden Größen in der Probe geschlossen.

Nachteilig bei dieser Ausführung ist insbesonders deren komplizierter Aufbau. Es ist besonders bei der Blutgasanalyse von Nachteil, wenn das Blut erst von einem Probennahmeelement, wie beispielsweise einer Kolbenspritze, in die Probenkammer gefüllt werden muß und von dieser erst nach einer weiteren Manipulation in den Meßkanal gelangt, da durch den möglichen Luftzutritt bei dieser Vorgangsweise eine Verfälschung der Blutgaswerte auftritt und reproduzierbare Messungen nur schwer möglich sind. Da der Probentransport lediglich durch Kapillarkräfte erfolgt, kann ein Vermischen der Kalibrierflüssigkeit mit der Probenflüssigkeit und eine dadurch verursachte Fehlmessung nicht ausgeschlossen werden. Diese Manipulationen bergen auch die Gefahr in sich, daß das Bedienungspersonal durch Blutproben infiziert wird.

Aufgabe der Erfindung ist es, diese Nachteile zu vermeiden und ein Einweg-Meßelement mit hoher Meßgenauigkeit, unabhängig von der Lagerung des Meßelementes anzugeben, wobei das Einweg-Meßelement einen für die Massenfertigung möglichst einfachen Aufbau aufweisen soll. Weiters soll ein Einweg-Meßelement vorgeschlagen werden, bei welchem der Probentransport von der Entnahme bis in den Meßbereich des Meßkanals möglichst kurz und direkt erfolgen soll.

Diese Aufgabe wird erfindungsgemäß dadurch gelöst, daß im Bereich der zweiten Öffnung ein Auffangbehälter im Meßelement angeordnet ist, welcher aus dem Meßkanal austretende Flüssigkeit aufnimmt, daß das erste Verschlußorgan mindestens zwei Stellungen aufweist, nämlich Absperrung und Verbindung des Meßkanals mit der ersten Öffnung und daß das zweite Verschlußorgan mindestens drei Stellungen aufweist, nämlich Absperrung, Verbindung des Meßkanals mit dem Auffangbehälter und Verbindung des Meßkanals mit der zweiten Öffnung.

Das Meßelement ist in seinem Inneren kanalförmig und besitzt an seinen beiden Enden Öffnungen, die als Kupplungen zum Anschluß des Analysegerätes bzw. des Probennahmeteiles ausgebildet sind. Vor der Messung sind beide Öffnungen durch die Verschlußorgane verschlossen und im Meßkanal befindet sich eine Lagerflüssigkeit, die die Sensoren vor unerwünschten Veränderungen schützt. Nach dem Anschluß des Analysegerätes an die erste Öffnung wird das erste Verschlußorgan betätigt, um eine Verbindung zwischen der Anschlußleitung des Analysegerätes und dem Meßkanal herzustellen. Gleichzeitig wird das zweite Verschlußorgan betätigt, um den Meßkanal mit dem Auffangbehälter zu verbinden. Der Probennahmeteil kann bereits an die zweite Öffnung angesteckt sein. Auf diese Weise kann durch Aufbringen eines Druckes am Analysegerät das Lagermedium aus dem Meßkanal in den Auffangbehälter verdrängt werden und ein Kalibriermedium in den Meßkanal eingebracht werden. In diesem Zustand erfolgt die Kalibrierung. Das Kalibriermedium kann ein wasserdampfgesättigtes Gasgemisch sein, welches einen Partialdruck von etwa 90 mm Hg O₂ und etwa 35 mm Hg CO₂ sowie ein Inertgas, vorzugsweise Stickstoff, enthält. Auf diese Weise ist eine Zweipunktkalibration möglich, welche bei Werten erfolgt, die den physiologischen Normalwerten sehr nahe kommen.

Wird das zweite Verschlußorgan in eine dritte Stellung gebracht, so wird der Meßkanal mit dem Probennahmeteil verbunden. Durch Umsteuerung des Analysegerätes kann nun die zu analysierende Probe, beispielsweise Blut, in den Meßkanal angesaugt werden. Nach Durchführung der Messung werden beide Verschlußorgane verschlossen, wodurch gewährleistet ist, daß auch nach der Entsorgung des Meßelementes die Probe im Inneren des Meßelementes verbleibt. Ein Kontakt des Bedienungspersonals mit der Probe wird auf diese Weise zuverlässig verhindert.

Bei der Verwendung von Sensoren, welche nicht kalibriert werden müssen, kann als Trennmedium beispielsweise Luft oder ein Inertgas verwendet werden.

In allen anderen Fällen ist vorgesehen, daß das Trennmedium ein Kalibriermedium ist. Vorteilhafterweise kann sich das Kalibriermedium in seiner Zusammensetzung vom Lagermedium unterscheiden, wobei es auch möglich ist, Gase bzw. Gasgemische für die Kalibrierung von Sensoren, z.B. CO₂- oder O₂-Sensoren, zu verwenden.

Da die entsprechenden Befüllungen und Entleerungen des Meßkanales nicht ausschließlich auf Kapillarkräften beruhen, sondern durch Druckunterschiede verursacht werden, die durch das Analysegerät aufgebracht werden, kann eine Vermischung von Lagermedium, Kalibriermedium und Probe weitgehend vermieden werden. Auf diese Weise wird eine genaue und reproduzierbare Messung gewährleistet.

Vorzugsweise kann jeweils ein Medium durch das nachfolgende verdrängt werden. Es ist im Prinzip möglich, daß das Lagermedium und/oder das Kalibriermedium jeweils durch das Einblasen von Luft aus dem Meßkanal verdrängt wird. Dies ist jedoch nicht erforderlich, wenn das Lagermedium direkt durch das Kalibriermedium und dieses direkt durch die Probe verdrängt wird.

Eine besonders hygienische und sichere Vorgangsweise besteht darin, daß nach der Messung die Öffnungen des Meßkanales verschlossen werden, worauf das Meßelement vom Analysegerät und vom Probennahmeteil abgekuppelt und entsorgt werden kann.

In einer bevorzugten Ausführungsvariante der Erfindung ist vorgesehen, daß im Bereich der ersten Öffnung ein Pufferbehälter vorgesehen ist und daß das erste Verschlußorgan mindestens eine weitere Stellung aufweist, in der der Meßkanal mit dem Pufferbehälter verbunden ist.

Weiters betrifft die Erfindung ein Meßelement mit einem Meßkanal mit mindestens einem darin angeordneten optischen oder elektrochemischen Sensor, einer ersten Öffnung zum Anschluß eines Analysegerätes, sowie einer zweiten Öffnung am anderen Ende des Meßkanals zum Anschluß eines Probennahmeteiles. Dieses Meßelement ist dadurch gekennzeichnet, daß ein gemeinsames Verschlußorgan für die beiden Öffnungen vorgesehen ist, welches drei Stellungen aufweist, wobei in der ersten Stellung dieses Verschlußorganes der Meßkanal beidseitig verschlossen ist, in der zweiten Stellung des Verschlußorganes ein Ende des Meßkanals mit der ersten Öffnung verbunden ist und das andere Ende des Meßkanals mit einem im Meßelement vorgesehenen Auffangbehälter für aus dem Meßkanal austretende Flüssigkeiten verbunden ist und in der dritten Stellung das eine Ende des Meßkanals mit einem im Meßelement vorgesehenen Pufferbehälter verbunden ist und das andere Ende des Meßkanals mit der zweiten Öffnung verbunden ist. Es wird eine Vereinfachung in der Herstellung und auch in der Bedienung des Meßelementes dadurch erreicht, daß lediglich ein einziger Schieber vorgesehen ist, der die entsprechende Verbindung herstellt. Der Meßkanal ist dabei im wesentlichen U-förmig ausgebildet, sodaß die beiden Enden des Meßkanals in unmittelbarer Nähe zueinander liegen.

Um eine mögliche Kontaminierung des Analysegerätes durch die Probe zu verhindern, ist vorgesehen, daß ein etwaiger Überschuß der Probe in den Pufferbehälter abgeleitet wird. In der weiteren Stellung des ersten Verschlußorganes kann dabei zusätzlich zur Verbindung des Meßkanals mit dem Pufferbehälter eine geeignete Verbindung des Meßkanals mit der ersten Öffnung vorgesehen sein, durch die der Meßkanal evakuiert werden kann. Die Konstruktion dieser Verbindung ist so ausgelegt, daß die Probe nicht hindurchdringen kann. Es ist gleichwohl auch möglich, den Unterdruck unmittelbar nach dem Kalibrieren in jener Stellung des ersten Verschlußorganes aufzubringen, in der der Meßkanal mit der ersten Öffnung verbunden ist.

Es ist im Sinne der Erfindung besonders vorteilhaft, wenn die Verschlußorgane als Schieber ausgebildet sind, die in Durchbrechungen des Meßelementes im wesentlichen quer zu dessen Längsachse verschiebbar sind, bzw. wenn die Verschlußorgane als Drehschieber ausgebildet sind, die in Durchbrechungen des Meßelementes drehbar aber axial unverschieblich gelagert sind. In beiden Fällen ist es möglich, die Schieber und das gesamte Meßelement besonders kostengünstig herzustellen, was für einen einmal zu verwendenden Bauteil sehr bedeutsam ist.

In einer Ausführungsvariante der Erfindung ist vorgesehen, daß das gemeinsame Verschlußorgan als Schieber ausgebildet ist, welcher in einer Führung des Meßelementes verschiebbar ist, wobei der Schieber einen Auffangbehälter aufweist, welcher vorzugsweise auch als Pufferbehälter dient.

Ein weiterer Vorteil dieser Ausführung liegt darin, daß diese Schieber durch entsprechende Stempel eines dazu geeigneten Analysegerätes automatisch betätigt werden können. Die Durchgänge in den Schiebern, die die entsprechenden Verbindungen herstellen, sind dabei in verschiedenen Ebenen übereinander angeordnet.

Der Auffangbehälter kann entweder ein variables Volumen aufweisen oder vorzugsweise nach außen hin belüftet sein.

Bei der Analyse kann mit einer besonders geringen Menge an Probenmaterial das Auslangen gefunden werden, wenn der Meßkanal als flache Kapillare ausgebildet ist.

Das Einweg-Meßelement kann erfindungsgemäß an der äußeren Oberfläche elektrische Kontaktflächen aufweisen, welche mit elektrochemischen Sensoren oder mit einem Temperaturmeßelement im Meßkanal in Verbindung stehen.

Eine weitere Ausführungsvariante der Erfindung sieht vor, daß der Meßkanal einen Temperatursensor aufweist, welcher auf eine Änderung der Temperatur durch eine Änderung seiner optischen Eigenschaften, vorzugsweise durch eine Änderung der Fluoreszenzabklingzeit, reagiert.

Während bei Einweg-Meßelementen mit optischen Sensoren transparente Bauteile für die Lichtführung vorhanden sein müssen, kann es auch für Meßelemente mit elektrochemischen Sensoren vorteilhaft sein, wenn der Meßkanal zur Messung intrinsischer, optischer Eigenschaften der Probe zumindest einen optisch durchlässigen Bereich aufweist. Das erfindungsgemäße Meßelement kann dann auch zur Messung der Eigenfluoreszenz, der Absorption, der Reflektivität oder des Brechungsindex der Probe herangezogen werden.

Im folgenden wird die Erfindung anhand der in den Zeichnungen dargestellten Ausführungsbeispiele näher erläutert. Es zeigen:
- Fig. 1: eine schematische Darstellung eines erfindungsgemäßen Meßelementes, das an das Analysegerät angeschlossen ist.
- Fig. 2: den Längsschnitt nach Linie II-II in Fig. 3,
- Fig. 3: eine Ansicht der unteren Hälfte des erfindungsgemäßen Meßgerätes,
- Fig. 4: einen Schnitt nach der Linie IV-IV in Fig. 2,
- Fig. 5: einen Schnitt des ersten Schiebers nach der Linie V-V in Fig. 6,
- Fig. 6: einen Schnitt nach der Linie VI-VI in Fig. 5,
- Fig. 7: einen Schnitt des Meßelementes nach der Linie VII-VII in Fig. 2,
- Fig. 8: einen Schnitt des zweiten Schiebers nach Linie VIII-VIII in Fig. 9,
- Fig. 9: einen Schnitt nach der Linie IX-IX in Fig. 8,
- Fig. 10: einen Schnitt des Meßelementes nach Linie X-X in Fig. 3 und
- Fig. 11a bis 11d: Darstellungen des erfindungsgemäßen Meßelementes entsprechend Fig. 3 in den einzelnen Schritten des Verfahrens,
- Fig. 12: eine weitere Ausführungsvariante eines erfindungsgemäßen Meßelementes im Schnitt nach Linie XII-XII in Fig. 13,
- Fig. 13: einen Schnitt nach der Linie XIII-XIII in Fig. 12,
- Fig. 14: einen Schnitt nach der Linie XIV-XIV in Fig. 13 und
- Fig. 15: einen Schnitt nach Linie XV-XV in Fig. 13,
- Fig. 16ad bis 16dc: zeigen die Stellungen der einzelnen Drehschieber der in den Fig. 12 bis 15 dargestellten Ausführungsvariante während der einzelnen Schritte des Verfahrens,
- Fig. 17: zeigt eine weitere Ausführungsvariante des erfindungsgemäßen Meßelementes im Schnitt nach Linie XVII-XVII in Fig. 19,
- Fig. 18: einen Schnitt nach Linie XVIII-XVIII in Fig. 19,
- Fig. 19: einen Schnitt nach Linie XIX-XIX in Fig. 17 und
- Fig. 20: einen Schnitt nach Linie XX-XX in Fig. 19. Die
- Fig. 16aa bis 16da: bzw.
- Fig. 16ab bis 16db: und

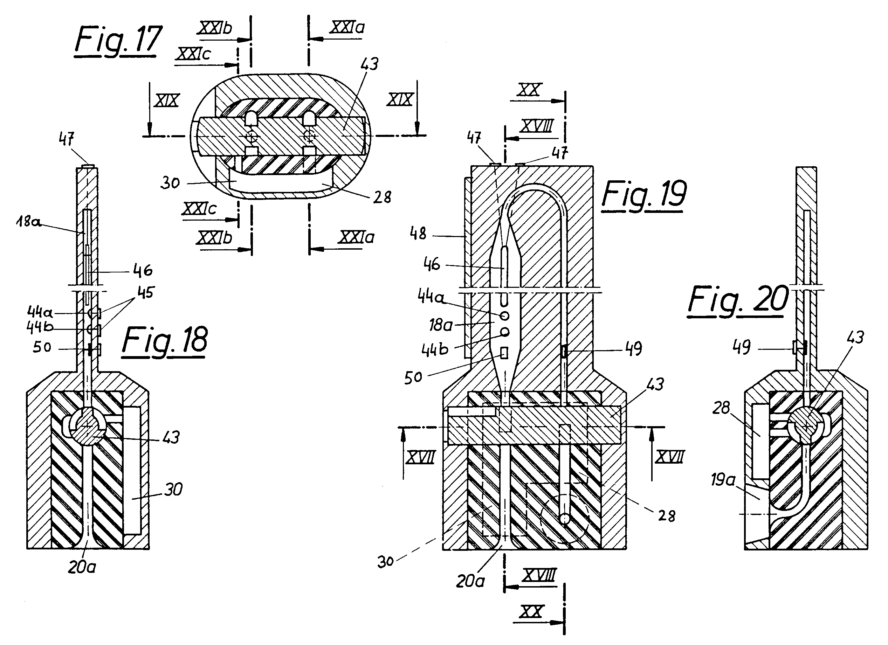

- Fig. 16ac bis 16dc: zeigen auch die Stellungen des Drehschiebers 43 der in den Fig. 17 bis 20 dargestellten Ausführungsvariante während der einzelnen Schritte des Verfahrens und zwar im Schnitt nach den Linien XXIa-XXIa bzw. XXIb-XXIb und XXIc-XXIc in Fig. 17.

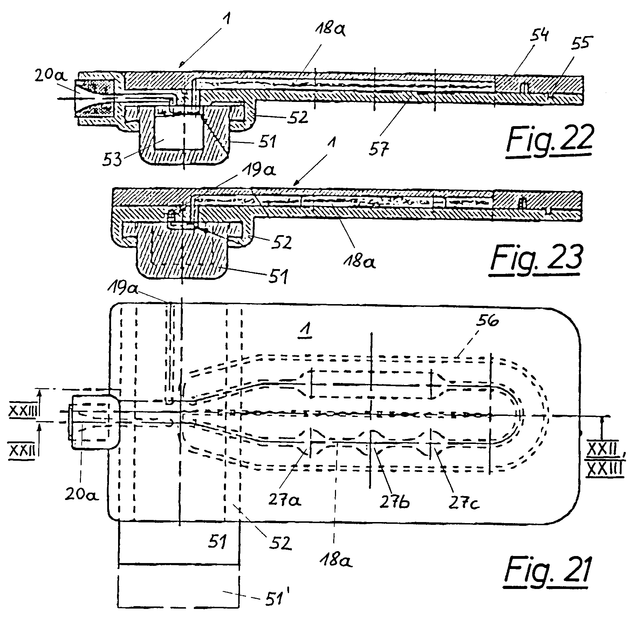

- Fig. 21: eine weitere Ausführungsvariante eines erfindungsgemäßen Meßelementes in Draufsicht,
- Fig. 22: einen Schnitt nach der Linie XXII-XXII in Fig. 21 und
- Fig. 23: einen Schnitt nach der Linie XXIII-XXIII in Fig. 21.

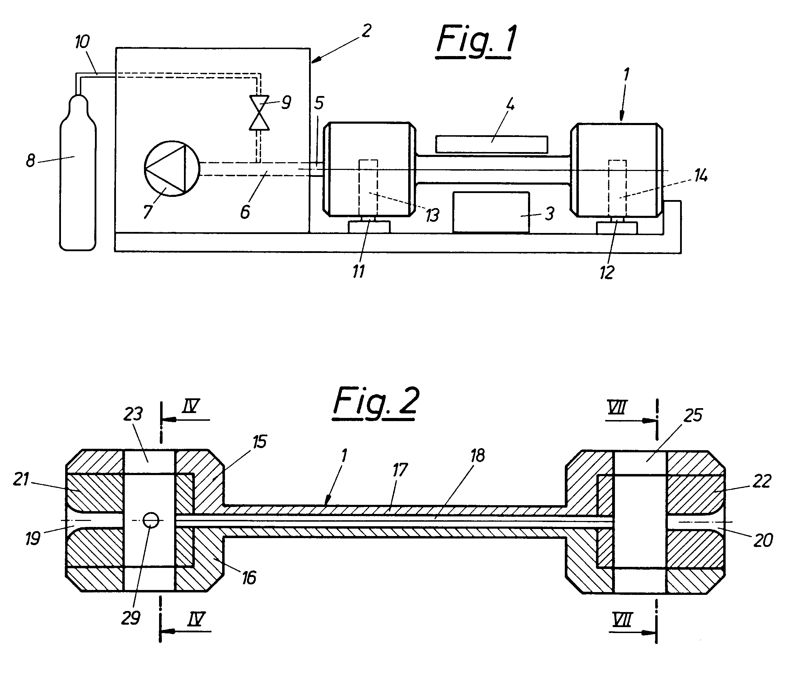

Wie aus Fig. 1 ersichtlich, ist ein Meßelement 1 mit einem Analysegerät 2 verbunden und wird von diesem gehalten. Das Analysegerät 2 weist eine Optik 3 zur Durchführung der erforderlichen Messungen auf. Weiters ist ein Thermostat 4 vorgesehen, um eine genau definierte Temperatur im Meßbereich einstellen zu können. Über einen Anschlußnippel 5 und eine Leitung 6 ist eine Pumpe 7 im Analysegerät 2 mit dem Meßelement 1 verbunden. Das Kalibriermedium wird aus einem Gasbehälter 8 entnommen und über eine mit einem Ventil 9 versehene Leitung 10 in die Leitung 6 eingespeist. Weiters sind am Analysegerät 2 Stempel 11 und 12 vorgesehen, mit denen der erste Schieber 24 bzw. der zweite Schieber 26 betätigt werden können.

Aus den Fig. 2 bis 4 ist ersichtlich, daß das Meßelement 1 aus einem oberen Gehäuseteil 15 und einem unteren Gehäuseteil 16 zusammengesetzt ist. Diese beiden Gehäuseteile 15 und 16 sind vorzugsweise möglichst identisch ausgeführt, sodaß das Meßelement 1 besonders leicht hergestellt werden kann. Das Meßelement 1 besteht aus einem Mittelteil 17, in dem der Meßkanal 18 angeordnet ist. An den beiden Enden des Meßelementes 1 ist die erste Öffnung 19 und die zweite Öffnung 20 zum Anschluß für das Analysegerät 2 bzw. den nicht dargestellten Probennahmeteil vorgesehen. Zur Ankopplung an das Gerät bzw. des Probennahmeteils sind an den Enden des Meßelementes 1 Einsätze 21 und 22 in entsprechenden Ausnehmungen angeordnet.

Im Meßkanal 18 sind optische Sensoren 27a, 27b und 27c angeordnet, mit deren Hilfe die Probe analysiert werden kann.

Anstelle der optischen Sensoren können jedoch auch elektrochemische Sensoren verwendet werden, wie beispielsweise anhand der Ausführungsvariante nach Fig. 18 und 19 dargelegt. Es ist auch möglich, einen der optischen Sensoren, z.B. 27c, zur Temperaturmessung heranzuziehen, wobei z.B. eine Indikatorsubstanz verwendet wird, deren Fluoreszenzabklingzeit temperaturabhängig ist.

Im Bereich der Öffnung 19 ist eine erste Durchbrechung 23 vorgesehen, in der ein erster Schieber 24 verschiebbar angeordnet ist. Die Durchbrechung 23 kreuzt den Meßkanal 18. Im Bereich der Öffnung 20 ist eine zweite Durchbrechung 25 vorgesehen, in der ein Schieber 26 verschiebbar angeordnet ist. Ein Pufferbehälter 28 im Bereich der ersten Öffnung 19 ist über einen Kanal 29 mit der ersten Durchbrechung 23 seitlich verbunden. Ein Auffangbehälter 30 im Bereich der zweiten Öffnung 20 ist über einen Kanal 31 mit der zweiten Durchbrechung 25 seitlich verbunden.

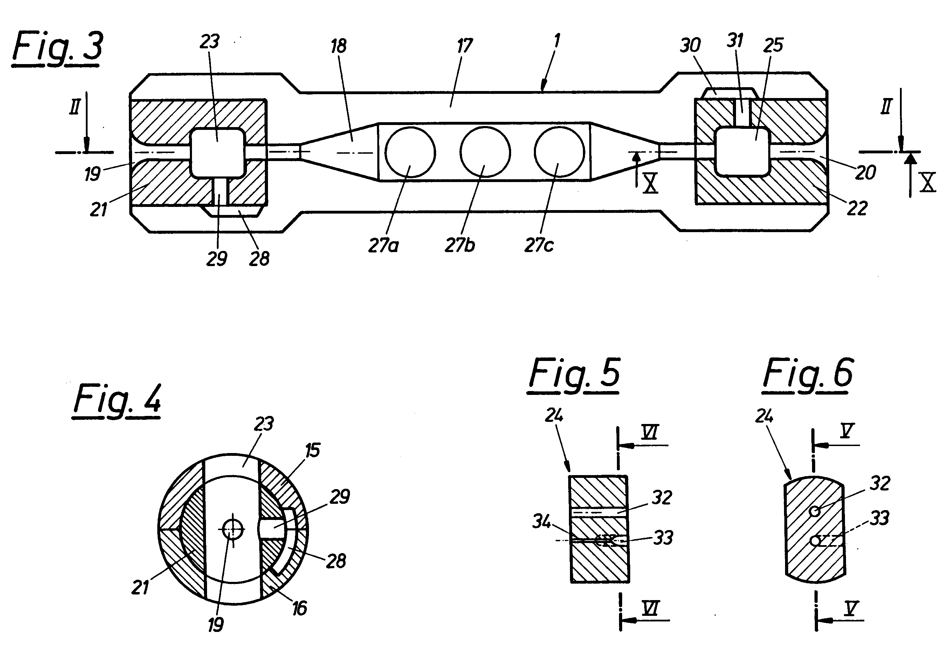

Wie aus den Fig. 5 und 6 zu ersehen ist, besitzt der erste Schieber 24 einen Kanal 32, der bei geeigneter Stellung des Schiebers 24 den Meßkanal 18 mit der ersten Öffnung 19 verbindet. Weiters besitzt der Schieber 24 einen weiteren Kanal 33, der bei geeigneter Stellung des Schiebers 24 den Meßkanal 18 mit dem Pufferbehälter 28 verbindet. Mit diesem Kanal 33 ist weiters ein dünner Saugkanal 34 verbunden, mit dem über die Öffnung 19 ein Unterdruck im Meßkanal 18 hergestellt werden kann.

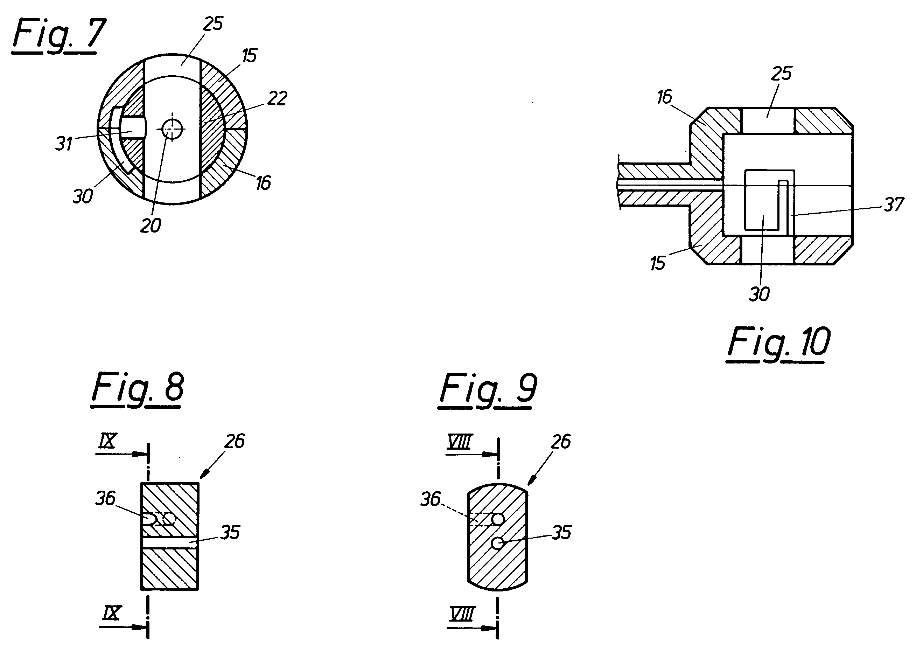

Der Schieber 26 (Fig. 7 bis 9) weist einen Kanal 35 auf, der in der geeigneten Stellung des Schiebers 26 den Meßkanal 18 mit der zweiten Öffnung 20 verbindet. Ein weiterer Kanal 36 verbindet in einer anderen Stellung des Schiebers 26 den Meßkanal 18 mit dem Kanal 31 und damit mit dem Auffangbehälter 30. Die Fig. 10 zeigt diesen Auffangbehälter 30 samt seiner Entlüftung 37. Durch entsprechende Konstruktion der Entlüftung 37 kann das gasförmige Kalibriermedium austreten, die Lagerflüssigkeit verbleibt jedoch im Auffangbehälter.

Im folgenden wird die Funktion des Meßelementes 1 anhand der Fig. 11a bis 11d näher erläutert.

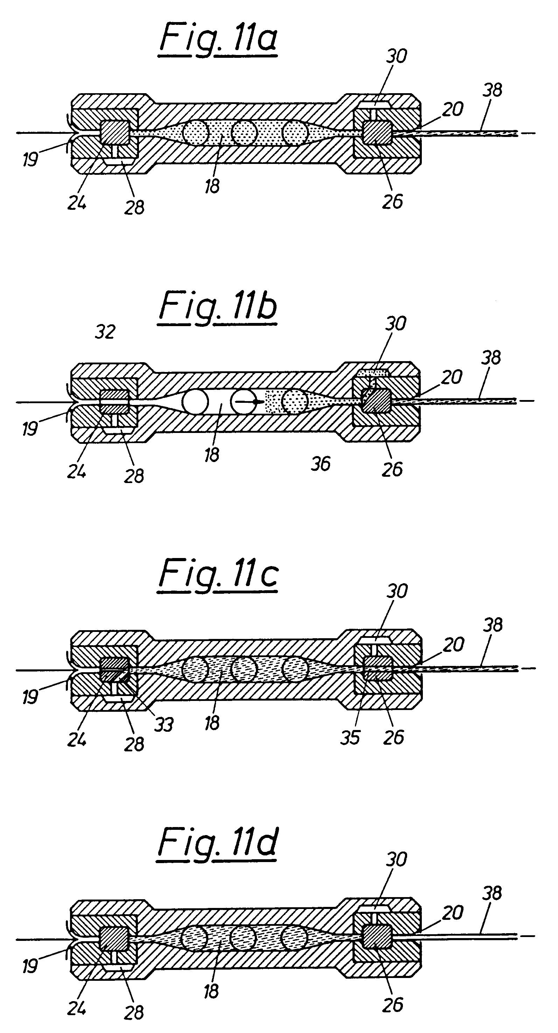

In der Fig. 11a ist der Zustand des Meßelementes 1 dargestellt, in dem es vertrieben und gelagert wird. Im Meßkanal 18 befindet sich das Lagermedium und der erste Schieber 24 und der zweite Schieber 26 sind geschlossen. Nach dem Anschluß des Meßelementes 1 an das Analysegerät 2 bzw. an den Probennahmeteil wird der erste Schieber 24 in eine Stellung gebracht, in der sein Kanal 32 den Meßkanal 18 mit der ersten Öffnung 19 verbindet (Fig. 11b). Der zweite Schieber 26 wird in eine Stellung gebracht, in der sein Kanal 36 den Meßkanal 18 des Meßelementes 1 mit dem Auffangbehälter 30 verbindet. Durch das Einbringen des Kalibriermediums oder Luft über die Öffnung 19 wird das Lagermedium aus dem Meßkanal 18 verdrängt und sammelt sich im Auffangbehälter 30. Das Kalibriergas kann durch die Entlüftung 37 entweichen, während das Lagermedium im Auffangbehälter 30 verbleibt. Nach Durchführung der Kalibriermessung wird der erste Schieber 24 in eine weitere Stellung gebracht (Fig. 11c), in der sein Kanal 33 den Meßkanal 18 des Meßelementes 1 mit dem Pufferbehälter 28 verbindet. Der Saugkanal 34 (Fig. 5) stellt eine Verbindung zur ersten Öffnung 19 her. Der zweite Schieber 26 wird in eine weitere Stellung gebracht, in der sein Kanal 35 eine Verbindung zwischen dem Meßkanal 18 und der zweiten Öffnung 20 herstellt. In dieser Stellung wird zunächst durch das Analysegerät 2 über den Saugkanal 34 ein Unterdruck im Meßkanal 18 des Meßelementes 1 hergestellt. Dadurch wird die zu analysierende Probe aus dem angedeuteten Probennahmeteil 38 angesaugt. Ein etwaiger Überschuß der Probe gelangt über den Kanal 33 des ersten Schiebers 24 in den Pufferbehälter 28. Nunmehr kann die eigentliche Messung durchgeführt werden.

Nach der Messung werden der erste Schieber 24 und der zweite Schieber 26 in eine weitere Stellung (Fig. 11d) gebracht, in der sie den Meßkanal 18 beidseitig verschließen. In dieser Stellung kann das Meßelement 1 vom Analysegerät 2 und dem Probennahmeteil 38 abgekuppelt und entsorgt werden.

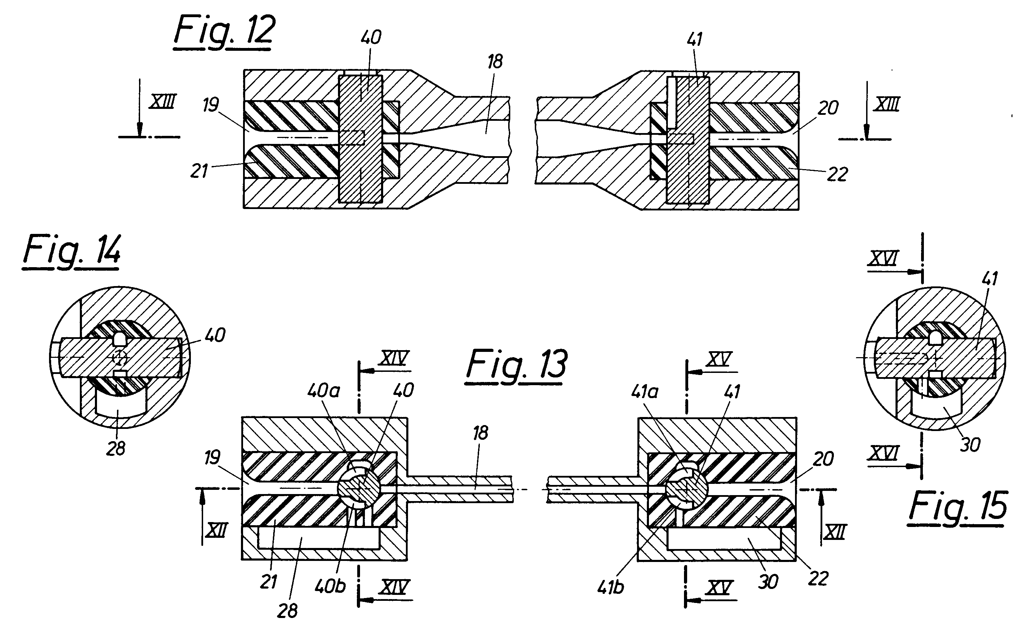

Das in den Fig. 12 bis 15 dargestellte Meßelement weist Drehschieber 40 und 41 auf, die an den beiden Enden des Meßkanales 18 drehbar aber axial unverschieblich angeordnet sind. Diese Drehschieber 40 und 41 besitzen Ausnehmungen 40a, 40b, 41a und 41b, die je nach Stellung der Drehschieber 40 und 41 eine Verbindung oder eine Trennung des Meßkanales 18 von der ersten Öffnung 19 bzw. der zweiten Öffnung 20 und dem Pufferbehälter 28 bzw. dem Auffangbehälter 30 ermöglichen.

In den Fig. 16aa bis 16dc sind die Stellungen der Drehschieber 40 bzw. 41 während der einzelnen Schritte des Verfahrens dargestellt. Dabei entsprechen die Fig. 16aa bis 16ac der Lager- bzw. Transportstellung, die Fig. 16ba bis 16bc der Stellung, in der das Lagermedium durch das Kalibriermedium verdrängt werden kann, die Fig. 16ca bis 16cc der Stellung, in der die Probe eingesaugt wird und die Fig. 16da bis 16dc die Stellung, in der das Meßelement entsorgt werden kann. Die Fig. 16ac bis 16dc entsprechen dabei einem Schnitt nach Linie XVI-XVI in Fig. 15. Aus den Fig. 16aa bis 16ac ist ersichtlich, daß der Meßkanal 18 beidseitig verschlossen ist. Somit kann das Lagermedium nicht verloren werden. In den Fig. 16ba bis 16bc ist der Drehschieber 40 um 90° im Uhrzeigersinn und der Schieber 41 um 90° im Gegenuhrzeigersinn gedreht. Das Lagermedium kann in dieser Stellung durch das Kalibriermedium in den Auffangbehälter 30 verdrängt werden. Über die Bohrung 42 ist eine Entlüftung des Auffangbehälters 30 vorgesehen.

In den Fig. 16ca bis 16cc sind die Drehschieber 40 und 41 um 180° gegenüber der vorigen Stellung verdreht. Die Probe kann nunmehr in den Meßkanal 18 eingesaugt werden, wobei das Kalibriermedium in den Pufferbehälter 28 eingesaugt wird.

In den Fig. 16da bis 16dc ist die ursprüngliche Stellung der Drehschieber 40 und 41 wiederum erreicht, sodaß die Probe 18 nicht aus dem Meßkanal entweichen kann und das Meßelement gefahrlos entsorgt werden kann.

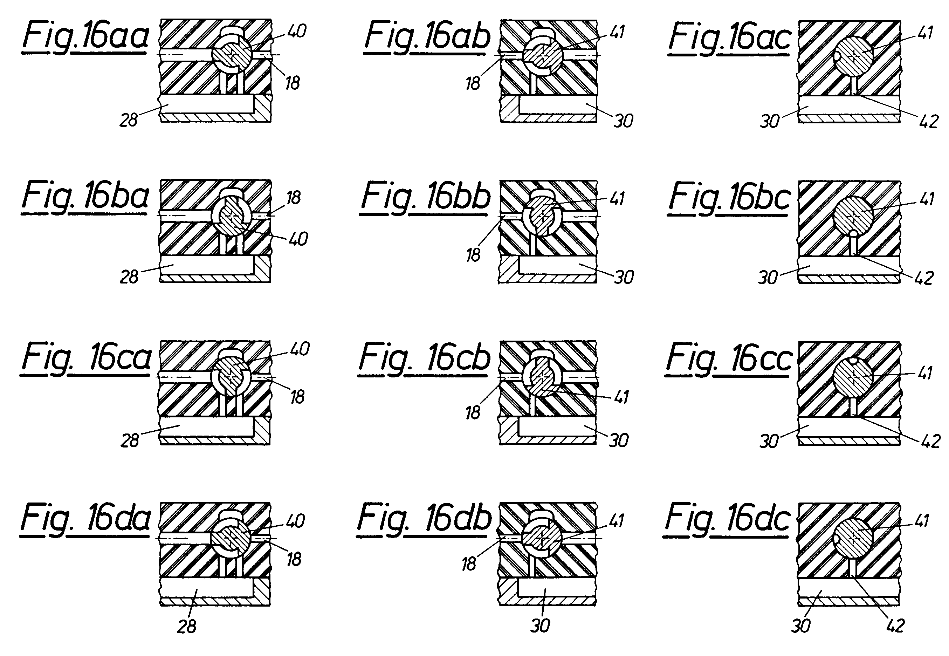

Bei der in den Fig. 17 bis 20 dargestellten Ausführungsvariante der Erfindung ist ein einziger Drehschieber 43 vorgesehen; der Meßkanal 18a ist U-förmig gebogen, sodaß sich die erste Öffnung 19a und die zweite Öffnung 20a an der selben Seite des Meßelementes befinden. Durch den Drehschieber 43 werden bei dieser Ausführungsvariante sowohl die Funktion des Drehschiebers 40 als auch des Drehschiebers 41 der in den Fig. 12 bis 15 dargestellten Ausführungsvariante erfüllt. Der Schnitt nach der Linie XXIa-XXIa in Fig. 17 entspricht dabei den Fig. 16aa bis 16da, der Schnitt nach der Linie XXIb-XXIb in Fig. 17 den Fig. 16ab bis 16db und der Schnitt nach der Linie XXIc-XXIc in Fig. 17 den Fig. 16ac bis 16dc. Für die Funktion dieser Ausführungsvariante gilt das zu Fig. 16 oben näher Ausgeführte entsprechend. Wie aus Fig. 19 ersichtlich, können in dieser Ausführungsvariante der Auffangbehälter 30 und der Pufferbehälter 28 zusammenfallen.

Wie anhand der Ausführungsvariante nach Fig. 17 bis 20 dargestellt, ist es auch möglich, elektrochemische Sensoren 44a, 44b im Meßkanal 18a vorzusehen, welche über elektrische Kontaktflächen 45 an der äußeren Oberfläche des Meßelementes kontaktiert werden.

Weiters ist es möglich, ein Temperaturmeßelement 46 im Meßkanal 18a vorzusehen, dessen Signale über Kontaktflächen 47 abgegriffen werden können, sobald das Meßelement in ein Analysegerät, entsprechend Fig. 1, eingesetzt wird. Gleiches gilt für eine Einrichtung zur Bestimmung der Leitfähigkeit, welche Elektroden 49, 50 aufweist, die in den Meßkanal 18a eintauchen.

Schließlich können Angaben, wie das Herstellungsdatum, die Sensorbestückung, Sensorkenndaten oder auch die Meßempfindlichkeit des Einweg-Meßelementes in einem Magnet- oder Streifencode, aber auch in einem vom Analysegerät abfragbaren Speicher-Chip 48, abgelegt sein.

Es ist natürlich möglich, auch für diese Ausführungsvariante optische Sensoren zu verwenden, bzw. für die vorher beschriebenen Varianten elektrochemische Sensoren, Temperaturmeßelemente sowie Codierelemente vorzusehen.

Bei der in den Fig. 21 bis 23 dargestellten Ausführungsvariante der Erfindung ist ein einziger Schieber 51 vorgesehen, welcher in einer Führung 52 des Meßelementes 1 verschoben werden kann. Der Meßkanal 18a ist auch hier im wesentlichen U-förmig gebogen, sodaß beide Öffnungen 19a und 20a mit einem Verschlußorgan betätigt werden können. Mit dem Schieber 51 (eine weitere Schieberposition ist mit 51' bezeichnet), werden sowohl die Funktionen des Schiebers 24 als auch jene des Schiebers 26, dargestellt in den Fig. 11a bis 11d, erfüllt. Bei dieser Ausführungsvariante befindet sich der Auffangbehälter 53 im Schieber 51 und dient auch gleichzeitig als Pufferbehälter.

Das Meßelement ist vorzugsweise zweiteilig aufgebaut, wobei der in Fig. 22 oben dargestellte Teil 54 einen Steg 55 aufweist, welcher beim Zusammenbauen in eine umlaufende Nut 56 des unteren Teiles 57 einrastet.

Der Schieber 51 ist in den Fig. 22 und 23 in einer Position dargestellt, in welcher der Meßkanal 18a des Meßelementes mit einem Lagermedium befüllt werden kann.

## Patentansprüche

1. Einweg-Meßelement, mit einem Meßkanal (18) mit mindestens einem darin angeordneten, optischen oder elektrochemischen Sensor (27a,b,c; 44a,b), einer ersten Öffnung (19) an einem Ende des Meßkanals (18) zum Anschluß eines Analysegerätes (2), einer zweiten Öffnung (20) am anderen Ende des Meßkanals (18) zum Anschluß eines Probennahmeteiles, einem ersten Verschlußorgan (24; 40) für die erste Öffnung (19) und einem zweiten Verschlußorgan (26; 41) für die zweite Öffnung (20), **dadurch gekennzeichnet**, daß im Bereich der zweiten Öffnung (20) ein Auffangbehälter (30) im Meßelement (1) angeordnet ist, welcher aus dem Meßkanal (18) austretende Flüssigkeit aufnimmt, daß das erste Verschlußorgan (24; 40) mindestens zwei Stellungen aufweist, nämlich Absperrung und Verbindung des Meßkanals (18) mit der ersten Öffnung (19) und daß das zweite Verschlußorgan (26; 41) mindestens drei Stellungen aufweist, nämlich Absperrung, Verbindung des Meßkanals (18) mit dem Auffangbehälter (30) und Verbindung des Meßkanals (18) mit der zweiten Öffnung (20).

2. Meßelement nach Anspruch 1, **dadurch gekennzeichnet**, daß im Bereich der ersten Öffnung (19) ein Pufferbehälter (28) vorgesehen ist und daß das erste Verschlußorgan (24; 40) mindestens eine weitere Stellung aufweist, in der der Meßkanal (18) mit dem Pufferbehälter (28) verbunden ist.

3. Meßelement nach Anspruch 1 oder 2, **dadurch gekennzeichnet**, daß die Verschlußorgane als Schieber (24, 26) ausgebildet sind, die in Durchbrechungen (23, 25) des Meßelementes (1) im wesentlichen quer zu dessen Längsachse verschiebbar sind.

4. Einweg-Meßelement, mit einem Meßkanal (18a) mit mindestens einem darin angeordneten optischen oder elektrochemischen Sensor (27a,b,c; 44a,b), einer ersten Öffnung (19a) zum Anschluß eines Analysegerätes, sowie einer zweiten Öffnung (20a) am anderen Ende des Meßkanals (18a) zum Anschluß eines Probennahmeteiles, **dadurch gekennzeichnet**, daß ein gemeinsames Verschlußorgan (43; 51) für die beiden Öffnungen (19a, 20a) vorgesehen ist, welches drei Stellungen aufweist, wobei in der ersten Stellung dieses Verschlußorganes (43; 51) der Meßkanal (18a) beidseitig verschlossen ist, in der zweiten Stellung des Verschlußorganes (43; 51) ein Ende des Meßkanals (18a) mit der ersten Öffnung (19a) verbunden ist und das andere Ende des Meßkanals (18a) mit einem im Meßelement (1) vorgesehenen Auffangbehälter (30; 53) für aus dem Meßkanal (18a) austretende Flüssigkeiten verbunden ist und in der dritten Stellung das eine Ende des Meßkanals (18a) mit einem im Meßelement (1) vorgesehenen Pufferbehälter (28; 53) verbunden ist und das andere Ende des Meßkanals (18a) mit der zweiten Öffnung (20a) verbunden ist.

5. Meßelement nach Anspruch 4, **dadurch gekennzeichnet**, daß das gemeinsame Verschlußorgan als Schieber (51) ausgebildet ist, welcher in einer Führung (52) des Meßelementes (1) verschiebbar ist.

6. Meßelement nach Anspruch 5, **dadurch gekennzeichnet**, daß der Schieber (51) einen Auffangbehälter (53) aufweist, welcher vorzugsweise auch als Pufferbehälter dient.

7. Meßelement nach einem der Ansprüche 1, 2 oder 4, **dadurch gekennzeichnet**, daß die Verschlußorgane als Drehschieber (40,41,43) ausgebildet sind, die in Durchbrechungen des Meßelementes drehbar aber axial unverschieblich gelagert sind.

8. Meßelement nach einem der Ansprüche 1 bis 7, **dadurch gekennzeichnet**, daß der Auffangbehälter (30; 53) belüftet ist.

9. Meßelement nach einem der Ansprüche 1 bis 8, **dadurch gekennzeichnet**, daß der Meßkanal (18,18a) als flache Kapillare ausgebildet ist.

10. Meßelement nach einem der Ansprüche 1 bis 9, **dadurch gekennzeichnet**, daß an der äußeren Oberfläche des Meßelementes elektrische Kontaktflächen (45) angeordnet sind, welche mit elektrochemischen Sensoren (44a,b) im Meßkanal (18a) in Verbindung stehen.

11. Meßelement nach einem der Ansprüche 1 bis 10, **dadurch gekennzeichnet**, daß an der äußeren Oberfläche des Meßelementes elektrische Kontaktflächen (47) angeordnet sind, welche mit einem Temperaturmeßelement (46) im Meßkanal (18a) in Verbindung stehen.

12. Meßelement nach einem der Ansprüche 1 bis 10, **dadurch gekennzeichnet**, daß der Meßkanal (18; 18a) einen Temperatursensor (27c) aufweist, welcher auf eine Änderung der Temperatur durch eine Änderung seiner optischen Eigenschaften, vorzugsweise durch eine Änderung der Fluoreszenzabklingzeit, reagiert.

13. Meßelement nach einem der Ansprüche 1 bis 12, **dadurch gekennzeichnet**, daß in den Meßkanal (18; 18a) Elektroden (49, 50) einer Einrichtung zur Bestimmung der Leitfähigkeit eintauchen.

14. Meßelement nach einem der Ansprüche 1 bis 13, **dadurch gekennzeichnet**, daß an der äußeren Oberfläche des Meßelementes ein Magnet- oder Streifencode oder ein Speicher-Chip (48) angeordnet ist.

15. Meßelement nach einem der Ansprüche 1 bis 14, **dadurch gekennzeichnet**, daß der Meßkanal (18, 18a) zur Messung von intrinsischen optischen Eigenschaften der Probe zumindest einen optisch durchlässigen Bereich aufweist.

## Claims

1. One-way measuring element, comprising a measuring channel (18) with at least one optical or electrochemical sensor (27a,b, c; 44a,b) located therein, a first opening (19) on one end of the measuring channel (18) for connection of an analyser (2), a second opening (20) on the other end of the measuring channel (18) for connection of a sample-taking part, and a first sealing element (24; 40) for the first opening (19) as well as a second sealing element (26; 41) for the second opening (20), **characterized in that** a collecting tank (30) receiving liquid issuing from the measuring channel (18) is located in the measuring element (1) in the area of the second opening (20), and that the first sealing element (24; 40) has at least two positions, i.e., closing the measuring channel (18) or connecting it to the first opening (19), and that the second sealing element (26; 41) has at least three positions, i.e. closing the measuring channel (18), connecting it to the collecting tank (30), or connecting it to the second opening (20).

2. Measuring element according to claim 1, **characterized in that** a buffer tank (28) is added in the area of the first opening (19), and that the first sealing element (24; 40) has at least one further position, in which the measuring channel (18) is connected with the buffer tank (28).

3. Measuring element according to claim 1 or 2, **characterized in that** the sealing elements are configured as sliding elements (24, 26), which slide in bores (23, 25) of the measuring element (1) and are movable essentially at right angles to the longitudinal axis of the measuring element (1).

4. One-way measuring element comprising a measuring channel (18a) with at least one optical or electrochemical sensor (21a,b,c; 44a,b) located therein, a first opening (19a) on one end of the measuring channel for connection of an analyser, and a second opening (20a) on the other end of the measuring channel (18a) for connection of a sample-taking part, **characterized in that** one common sealing element (43; 51) is used for both openings (19a, 20a), which has three positions, the first position of this sealing element (43; 51) closing the measuring channel (18a) on both ends, the second position connecting one end of the measuring channel (18a) to the first opening (19a) and the other end of the measuring channel (18a) to a collecting tank (30; 53) provided in the measuring element (1) for liquids issuing from the measuring channel (18a), and the third position of the sealing element (43; 51) connecting one end of the measuring channel (18a) to a buffer tank (28; 53) provided in the measuring element (1) and the other end of the measuring channel (18a) to the second opening (20a).

5. Measuring element according to claim 4, **characterized in that** the joint sealing element is configured as a sliding element (51), which is shifted in a guide (52) of the measuring element (1).

6. Measuring element according to claim 5, **characterized in that** the sliding element (51) is provided with a collecting tank (53), which preferably acts as a buffer tank as well.

7. Measuring element according to any of claims 1, 2, 4, **characterized in that** the sliding elements are configured as rotary valves (40, 41, 43), which may be rotated in bores of the measuring element, but cannot be shifted axially.

8. Measuring element according to any of claims 1 to 7, **characterized in that** the collecting tank (30; 53) has a vent.

9. Measuring element according to any of claims 1 to 8, **characterized in that** the measuring channel (18, 18a) is configured as a flat capillary.

10. Measuring element according to any of claims 1 to 9, **characterized in that** electric contact surfaces (45) are provided on the outer surface of the measuring element, which are connected to electrochemical sensors (44a,b) in the measuring channel (18a).

11. Measuring element according to any of claims 1 to 10, **characterized in that** electric contact surfaces (47) are provided on the outer surface of the measuring element, which are connected to a thermometer element (46) in the measuring channel (18a).

12. Measuring element according to any of claims 1 to 10, **characterized in that** the measuring channel (18; 18a) has a temperature sensor (27c) responding to a change in temperature by a change in its optical properties, preferably by a change in its fluorescence decay time.

13. Measuring element according to any of claims 1 to 12, **characterized in that** electrodes (49, 50) of a device for determining conductivity dip into the measuring channel (18; 18a).

14. Measuring element according to any of claims 1 to 13, **characterized in that** the outer surface of the measuring element is provided with a magnetic code or bar code or a memory chip (48).

15. Measuring element according to any of claims 1 to 14, **characterized in that** the measuring channel (18, 18a) has at least one optically transparent zone for detecting intrinsic optical properties of the sample.

## Revendications

1. Elément de mesure à une voie, avec un canal de mesure (18) ayant au moins un capteur (27a, b, c ; 44a, b) optique ou électrochimique placé à l'intérieur, un premier orifice (19) a une extrémité du canal de mesure (18) pour le raccordement à un appareil d'analyse (2), une deuxième ouverture (20) à l'autre extrémité du canal de mesure (18) pour le raccordement à une pièce de prise des échantillons, un premier obturateur (24 ; 40) pour le premier orifice (19) et un deuxième obturateur (26 ; 41) pour le deuxième orifice (20), élément caractérisé en ce que dans la zone de la deuxième ouverture (20) est disposé un espace collecteur (30) dans l'élément de mesure (1), qui reçoit du liquide provenant du canal de mesure (18), en ce que le premier obturateur (24 ; 40) a au moins deux positions, à savoir fermeture et liaison du canal de mesure (18) avec le premier orifice (19) et en ce que le deuxième obturateur (26 ; 41) a au moins trois positions, à savoir fermeture, liaison du canal de mesure (18) avec l'espace collecteur (30) et liaison du canal de mesure (18) avec le deuxième orifice (20).

2. Elément de mesure selon la revendication 1, caractérisé en ce que dans la zone du premier orifice (19) est prévu un réservoir tampon (28) et en ce que le premier obturateur (24, 40) a au moins une autre position, dans laquelle le canal de mesure (18) est relié au réservoir tampon (28).

3. Elément de mesure selon las revendications 1 ou 2, caractérisé en ce que les obturateurs sont des robinets-vannes (24, 26) à tiroir qui sont mobiles dans des découpes (23, 25) de l'élément de mesure (1) essentiellement transversalement à son axe longitudinal.

4. Elément de mesure à une voie, avec un canal de mesure (18a) ayant au moins un capteur (27a, b, c ; 44a, b) optique ou électrochimique placé dedans, un premier orifice (19a) pour connecter un appareil d'analyse, ainsi qu'un deuxième orifice (20a) a l'autre extrémité du canal de mesure (18a) pour raccorder une pièce de prise des échantillons, élément caractérisé en ce qu'un obturateur commun (43 ; 51) pour les deux orifices (19a, 20a) est prévu, avec trois positions ; dans la première position de cet obturateur (43 ; 51) le canal de mesure (18a) est fermé des deux côtés, dans la deuxième position de l'obturateur (43 ; 51) une extrémité du canal de mesure (18a) se trouve reliée au premier orifice (19a) et l'autre extrémité du canal de mesure (18a) est reliée à un espace collecteur (30 ; 53) prévu dans l'élément de mesure pour des liquides provenant du canal de mesure (18a) et dans la troisième position, l'une des extrémités du canal de mesure (18a) est reliée à un réservoir tampon (28 ; 53) prévu dans l'élément de mesure (1) et l'autre extrémité du canal de mesure (18a) est reliée au deuxième orifice (20a).

5. Elément de mesure selon la revendication 4, caractérisé en ce que l'obturateur commun est réalisé sous forme de tiroir (51), qui peut coulisser dans un guidage (52) de l'élément de mesure (1).

6. Elément de mesure selon la revendication 5, caractérisé en ce que le tiroir (51) comprend un collecteur (53) qui sert de préférence de réservoir tampon.

7. Elément de mesure selon une des revendications 1, 2 ou 4, caractérisé en ce que les obturateurs sont réalisés sous forme de tiroir rotatif (40, 41, 43), qui sont montés mobiles en rotation , mais non déplaçables axialement dans des découpes de l'élément de mesure.

8. Elément de mesure selon une des revendications 1 à 7, caractérisé en ce que l'espace collecteur (30 ; 53) est ventilé.

9. Elément de mesure selon une des revendications 1 à 8, caractérisé en ce que le canal de mesure (18, 18a) est réalisé en tube capillaire plat.

10. Elément de mesure selon une des revendications 1 à 9, caractérisé en ce que sur la surface extérieure de l'élément de mesure sont disposées des surfaces de contact électriques (45) qui sont reliées à des capteurs électrochimiques (44a, b) situés dans le canal de mesure (18a).

11. Elément de mesure selon une des revendications 1 à 10, caractérisé en ce que sur la surface extérieure de l'élément de mesure, sont disposées des surfaces de contact électrique (47) qui sont reliées à un élément de mesure de la température (46) situé dans le canal de mesure (18a).

12. Elément de mesure selon une des revendications 1 à 10, caractérisé en ce que le canal de mesure (18 ; 18a) comporte un capteur de température (27c), qui réagit à une variation de la température par une modification de ses propriétés optiques, de préférence par une modification de la durée de déclin de la fluorescence.

13. Elément de mesure selon une des revendications 1 à 12, caractérisé en ce que des électrodes (49, 50) d'un dispositif de détermination de la conductibilité sont immergées dans le canal de mesure (18 ; 18a).

14. Elément de mesure selon une des revendications 1 à 13, caractérisé en ce que sur la surface extérieure de l'élément de mesure est placé un code magnétique ou à bandes, ou bien une puce à mémoire (48).

15. Elément de mesure selon une des revendications 1 à 14, caractérisé en ce que le canal de mesure (18, 18a) comprend pour la mesure des propriétés optiques intrinsèques de l'échantillon, au moins un domaine de transparence optique.
